# EUROPEAN PATENT APPLICATION

(11) **EP 3 889 247 A1**
(43) Date of publication of application: **06.10.2021**
(21) Application number: 19888944.6
(22) Date of filing: 29.11.2019
(51) Int. Cl.: C12M 1/00, C12M 1/34, C12N 5/071, C12Q 1/02

(54) **PIGMENTATION SKIN MODEL AND METHOD FOR PRODUCING SAME, AND METHOD FOR EVALUATING FACTOR FOR TREATING OR PREVENTING PIGMENTATION OF SKIN**

(30) Priority: 30.11.2018 JP 2018226024
(71) Applicant: Shiseido Company, Ltd., Chuo-ku Tokyo 104-0061 (JP)
(72) Inventor: YAMADA, Shoko, Tokyo 104-0061 (JP); KOIDE, Sayaka, Tokyo 104-0061 (JP); TAKAGI, Masaya, Tokyo 104-0061 (JP); SOMA, Tsutomu, Tokyo 104-0061 (JP); FUKUMURA, Kenta, Tokyo 104-0061 (JP)
(74) Representative: Cabinet Beau de Loménie
(86) International application number: PCT/JP2019/046886
(87) International publication number: WO 2020/111265

(57) **Abstract**

The present invention provides a pigmentation skin model that comprises: a first cell group containing fibroblasts damaged by light irradiation, said first cell group being seeded on a first cell culture substratum; and a second cell group containing melanocytes and keratinocytes, said second cell group being applied onto the first cell group. The present invention also provides a method for producing the pigmentation skin model, and a method for evaluating a factor for treating or preventing pigmentation of the skin, said method comprising using the pigmentation skin model.

## Description

### FIELD

The present invention relates to a pigmentation skin model and to a method for producing it. The invention also relates to a method for evaluating factors for treatment or prevention of skin pigmentation using the pigmentation skin model.

### BACKGROUND

The skin is an organ that covers the surface of the body, separating the interior of the body from its exterior. Skin functions as a physical barrier and protects the interior of the body from dryness and infiltration of hazardous substances, thus performing an indispensable role for life maintenance.

The skin of higher vertebrates primarily consists of the epidermis, dermis and subcutaneous tissue layers, in that order from the outermost layer. The epidermis is composed mainly of cells known as keratinocytes, the keratinocytes dividing at the deepest part (basal lamina) of the epidermis while migrating to the surface and differentiating toward the upper layer into the stratum spinosum, granular layer and stratum corneum, eventually being shed as old skin.

Melanocytes are present in the basal lamina of the epidermis, with melanin being produced by melanosomes inside the melanocytes. The melanin that is produced is taken up into the surrounding keratinocytes. After being taken up, the melanin migrates to the stratum corneum with turnover of keratinocytes, being discharged out of the body over a period of about 40 days.

It is thought that pigmentation of skin, such as skin spots and freckles, is caused by overproduction of melanin by melanocytes due to hormone imbalance, ultraviolet ray exposure or local inflammation, and by deposition of melanin granules inside keratinocytes in the basal epidermal layer. Methods and agents (skin whiteners) for treatment or prevention of pigmentation such as senile pigmentation spots have been developed, but these have not provided the anticipated effects or have only exhibited temporary effects, resulting in relapse without continual use, and therefore an ongoing demand exists to develop new methods of treatment and prevention, and new therapeutic agents.

In light of this situation, pigmentation skin models are recently being developed and used which simulate skin structure and function, as substitutes for conventional animal models in order to develop new methods of treatment (PTL 1, for example).

### [CITATION LIST]

### [PATENT LITERATURE]

[PTL 1] Japanese Patent Publication No. 6113393

### SUMMARY

### [TECHNICAL PROBLEM]

Conventional pigmentation skin models have had varying levels of pigmentation between prepared models and have been difficult to culture in a stable manner for prolonged periods. This is the case because the method of preparation involves directly seeding an epidermal cell group that includes keratinocytes or melanocytes onto the dermis model, causing the quality of the dermis model to have a major effect on formation of the epidermis layer, while procedures such as separation of the dermis model and epidermis model for alteration of the conditions, or transferring of the epidermis model to a different dermis model, cannot be carried out during the course of maintenance culture of the pigmentation skin model, thereby restricting the scope of possible experimentation. It is therefore an object of the invention to provide a novel pigmentation skin model that can be stably cultured for long periods, and a method for its production, as well as to allow the pigmentation skin model to have the conditions altered for the dermis model or epidermis model alone, or to have the dermis model and epidermis model freely transferred, during the course of maintenance culturing. It is also another object of the invention to provide a method for evaluating factors for treatment or prevention of skin pigmentation using the novel pigmentation skin model.

### [SOLUTION TO PROBLEM]

As a result of avid research, the present inventors have found that it is possible to develop a pigmentation skin model that can be stably cultured for long periods by applying, on a first cell group that includes fibroblasts that have suffered damage by photoirradiation, a second cell group that includes melanocytes and keratinocytes. Specifically, the present invention encompasses the following inventions.

[1] A pigmentation skin model comprising:
   a first cell group that includes fibroblasts that have been damaged by photoirradiation, seeded on a first cell culturing substrate; and
   a second cell group that includes melanocytes and keratinocytes, applied onto the first cell group.
[2] The pigmentation skin model according to [1], wherein the fibroblasts that have been damaged by photoirradiation are fibroblasts that have been damaged by irradiation of ultraviolet light in the presence of a photosensitizer.
[3] The pigmentation skin model according to [2], wherein the photosensitizer is selected from the group consisting of psoralen, NAD, riboflavin, tryptophan, folic acid, porphyrin, methylene blue and thiol group-protected gold nanoclusters (AUxSRy).
[4] The pigmentation skin model according to any one of [1] to [3], wherein the photoirradiation is photoirradiation with UVA.
[5] The pigmentation skin model according to any one of [1] to [4], wherein the second cell group is a second cell group seeded onto a second cell culturing substrate having a porous membrane.
[6] The pigmentation skin model according to any one of [1] to [5], wherein the first cell culturing substrate is a cell culturing substrate having a porous membrane.
[7] The pigmentation skin model according to any one of [1] to [6], wherein the first cell group is a first cell group seeded together with a hydrogelling agent.
[8] The pigmentation skin model according to [7], wherein the hydrogelling agent is selected from the group consisting of collagen, gelatin, hyaluronate, hyaluronan, fibrin, alginate, agarose, chitosan, chitin, cellulose, pectin, starch, laminin, fibrinogen/thrombin, fibrillin, elastin, gum, cellulose, agar, gluten, casein, albumin, vitronectin, tenascin, entactin/nidogen, glycoproteins, glycosaminoglycan, poly(acrylic acid) and its derivatives, poly(ethylene oxide) and its copolymers, poly(vinyl alcohol), polyphosphazene, Matrigel, and combinations of the foregoing.
[9] A method for producing a pigmentation skin model, wherein the method comprises:
   (1) culturing a first cell group that includes fibroblasts that have been damaged by photoirradiation,
   (2) culturing the first cell group obtained in step (1) on a first cell culturing substrate, and
   (3) applying a second cell group that includes melanocytes and keratinocytes on the first cell group obtained in step (2), and culturing it.
[10] The method according to [9], wherein the fibroblasts that have been damaged by photoirradiation are fibroblasts that have been damaged by irradiation of ultraviolet light in the presence of a photosensitizer.
[11] The method according to [10], wherein the photosensitizer is selected from the group consisting of psoralen, NAD, riboflavin, tryptophan, folic acid, porphyrin, methylene blue and thiol group-protected gold nanoclusters (AUxSRy).
[12] The method according to any one of [9] to [11], wherein the photoirradiation is photoirradiation with UVA.
[13] The method according to any one of [9] to [12], wherein the second cell group is a second cell group seeded onto a second cell culturing substrate having a porous membrane.
[14] The method according to any one of [9] to [13], wherein the first cell culturing substrate is a cell culturing substrate having a porous membrane.
[15] The method according to any one of [9] to [14], wherein the first cell group is a first cell group seeded together with a hydrogelling agent.
[16] The method according to [15], wherein the hydrogelling agent is selected from the group consisting of collagen, gelatin, hyaluronate, hyaluronan, fibrin, alginate, agarose, chitosan, chitin, cellulose, pectin, starch, laminin, fibrinogen/thrombin, fibrillin, elastin, gum, cellulose, agar, gluten, casein, albumin, vitronectin, tenascin, entactin/nidogen, glycoproteins, glycosaminoglycan, poly(acrylic acid) and its derivatives, poly(ethylene oxide) and its copolymers, poly(vinyl alcohol), polyphosphazene, Matrigel, and combinations of the foregoing.
[17] The method according to any one of [9] to [16], wherein the step (2) is carried out in the presence of ascorbic acid, an ascorbic acid derivative or salt thereof.
[18] A pigmentation skin model obtained by the method according to any one of [9] to [17].
[19] A method for evaluating factors for treatment or prevention of skin pigmentation, wherein the method comprises:
   (1) applying a candidate factor to a pigmentation skin model according to any one of [1] to [8] or [18], and culturing it, and
   (2) evaluating the treatment or prevention effect of the candidate factor, using the extent of pigment production and/or pigmentation in the pigmentation skin model as an indicator.
[20] A method for evaluating factors for treatment or prevention of skin pigmentation, wherein the method comprises:
   (1) culturing a first cell group that includes fibroblasts that have been damaged by photoirradiation,
   (2) culturing the first cell group obtained in step (1) on the first cell culturing substrate,
   (3) applying a second cell group that includes melanocytes and keratinocytes onto the first cell group obtained in step (2) and culturing it,
   (4) transferring the second cell group obtained in step (3) onto a substrate containing a candidate factor, and culturing it, and
   (5) evaluating the treatment or prevention effect of the candidate factor, using the extent of pigment production and/or pigmentation in the second cell group as an indicator.
[21] The method according to [20], wherein the candidate factor is fibroblasts.

### [ADVANTAGEOUS EFFECTS OF INVENTION]

According to the invention it is possible to provide a pigmentation skin model that can be stably cultured for long periods, and which can be used to search for and evaluate factors for treatment or prevention of skin pigmentation.

### BRIEF DESCRIPTION OF DRAWINGS

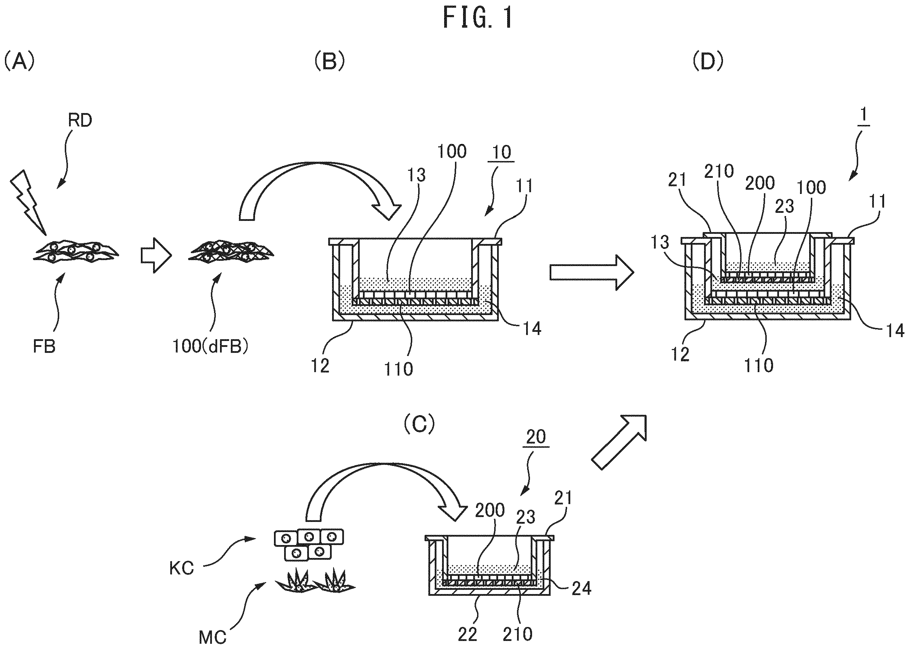

Fig. 1 is a schematic diagram showing a method for producing a pigmentation skin model according to an embodiment of the invention. The constituent elements are shown mainly in cross-sectional form.

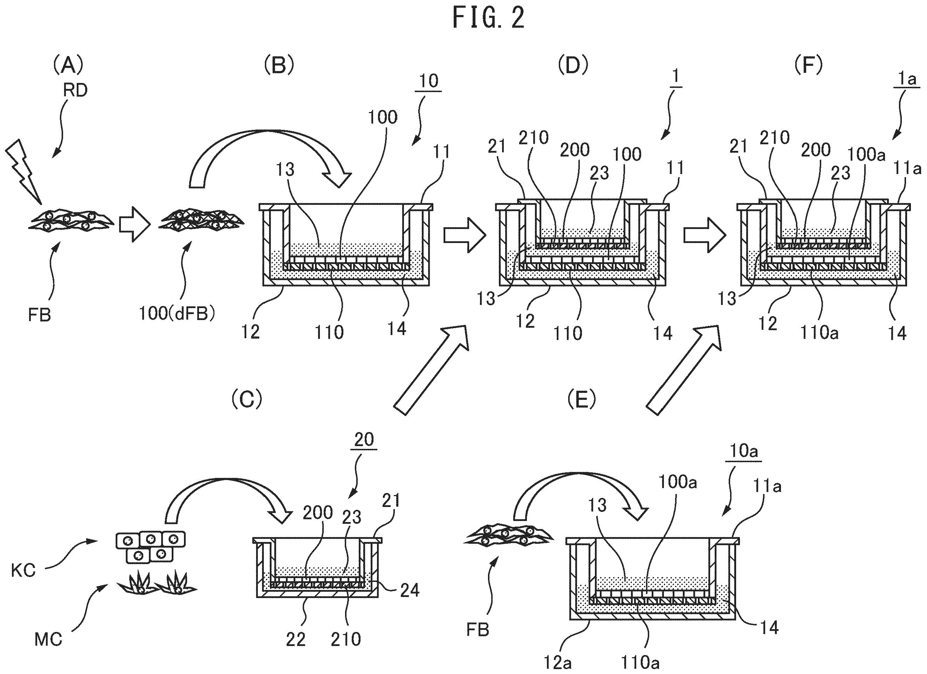

Fig. 2 is a schematic diagram showing a method for producing a pigmentation skin model according to an embodiment of the invention. The constituent elements are shown mainly in cross-sectional form.

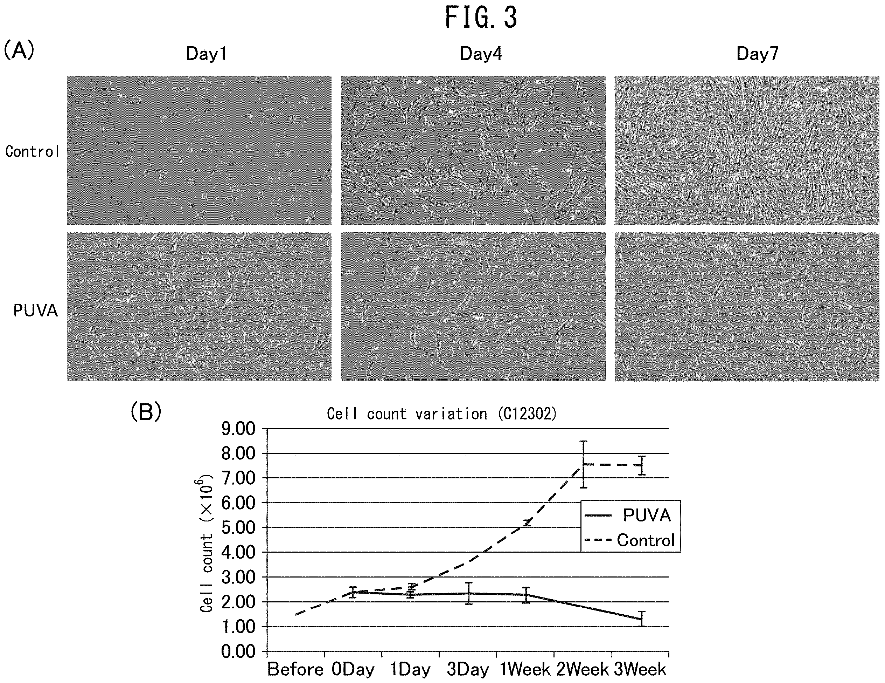

Fig. 3 shows differences in proliferative ability of fibroblasts treated and not treated with PUVA. (A) Image of monolayer cultured fibroblasts either treated with PUVA or not treated (control) (days 1, 4 and 7 of culturing). (B) Proliferation graph of fibroblasts after PUVA treatment.

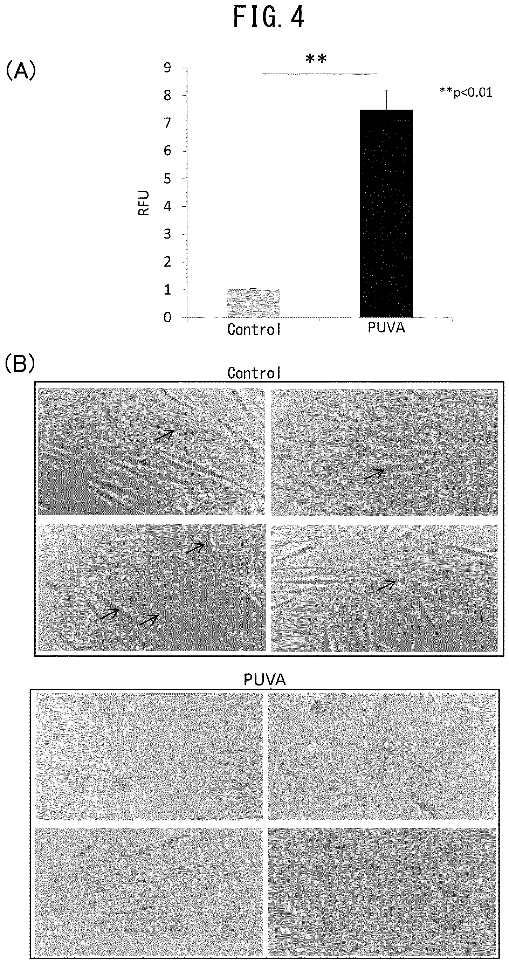

Fig. 4 shows differences in senescence-associated factor SA-β-gal production levels in fibroblasts either treated with PUVA or untreated (control). (A) Enzyme activity values of SA-β-gal in monolayer cultured fibroblast lysates. (B) SA-β-gal stained images. The SA-β-gal positive cells are stained blue.

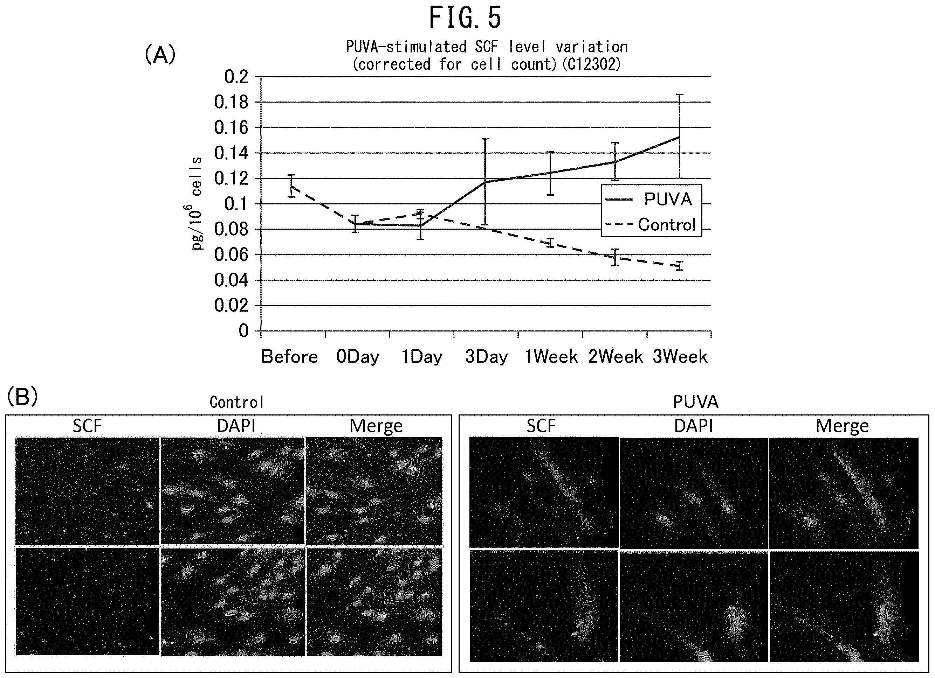

Fig. 5 shows differences in production levels of the melanin production promoting factor SCF (Stem Cell Factor) in fibroblasts either treated with PUVA or untreated (control). (A) Graph showing variation in SCF production with time. (B) SCF staining with SCF antibody, nuclear staining with DAPI, and fused (merged) image of SCF staining and nuclear staining (2 each).

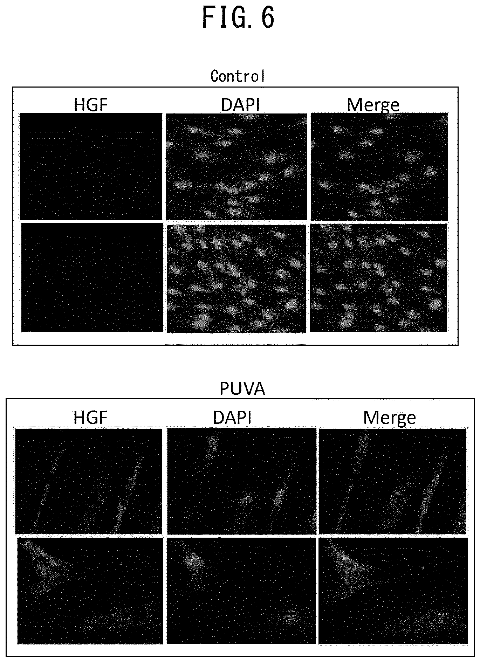

Fig. 6 shows differences in production levels of the melanin production promoting factor HGF (Hepatocyte Growth Factor) in fibroblasts either treated with PUVA or untreated (control). (A) HGF staining with HGF antibody, nuclear staining with DAPI, and fused (merged) image of HGF staining and nuclear staining (2 each).

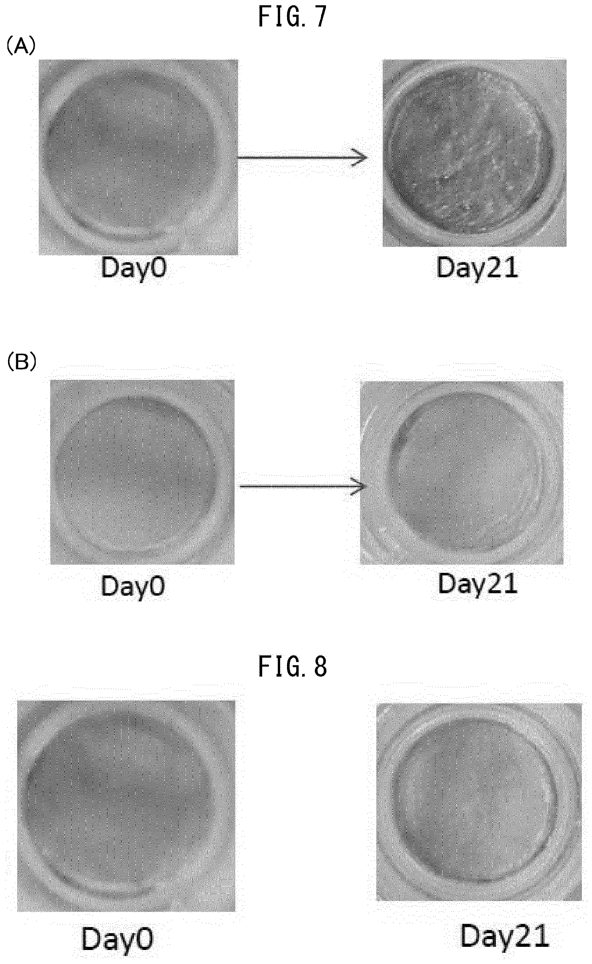

Fig. 7 is a diagram showing pigmentation of a pigmentation skin model either treated or not treated with PUVA. (A) Epidermis model image, for a pigmentation skin model treated with PUVA (day 0 and day 21 of culturing). (B) Epidermis model image, for a pigmentation skin model not treated with PUVA (day 0 and day 21 of culturing).
Fig. 8 is an image of an epidermis model, prepared by co-culturing an epidermis model for approximately 10 days with a PUVA-treated dermis model, and then transferring onto a PUVA-untreated dermis model and culturing for approximately 10 days.

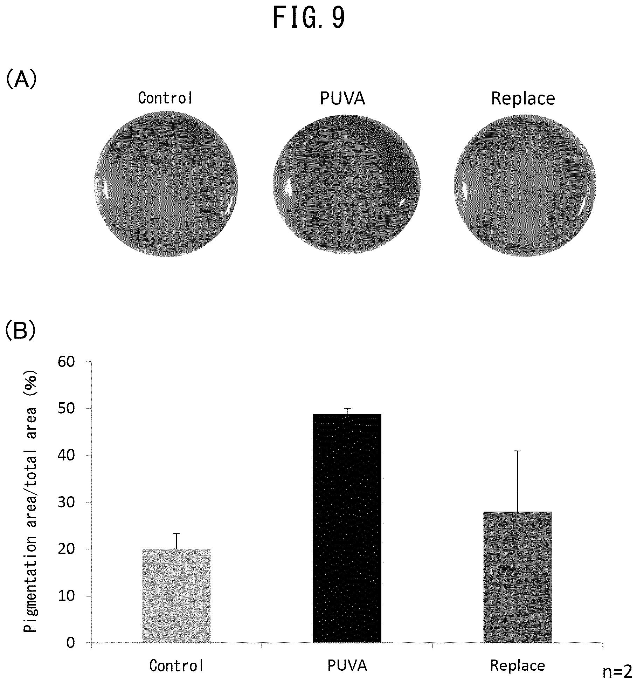

Fig. 9 shows pigmentation in a pigmentation skin model either treated with PUVA or not treated (control), and a PUVA-treated pigmentation epidermis model, transferred onto a PUVA-untreated dermis model and cultured (Replace). (A) Image of epidermis model photographed after fixing the observation conditions in the bright field of a phase contrast microscope. (B) Graph showing binarization of image (A), with quantification of the pigmentation area percentage.

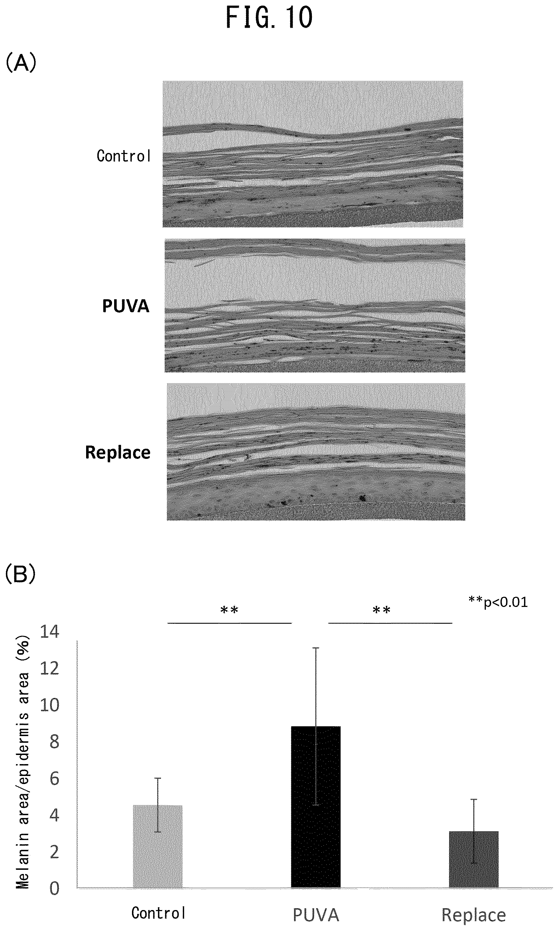

Fig. 10 shows melanin granules in a pigmentation skin model either treated with PUVA or not treated (control), and a PUVA-treated pigmentation epidermis model, transferred onto a PUVA-untreated dermis model and cultured (Replace). (A) Staining of melanin granules in epidermis model slice by Fontana-Masson staining. (B) Graph showing binarization of image (A), with quantification of the melanin granule area percentage.

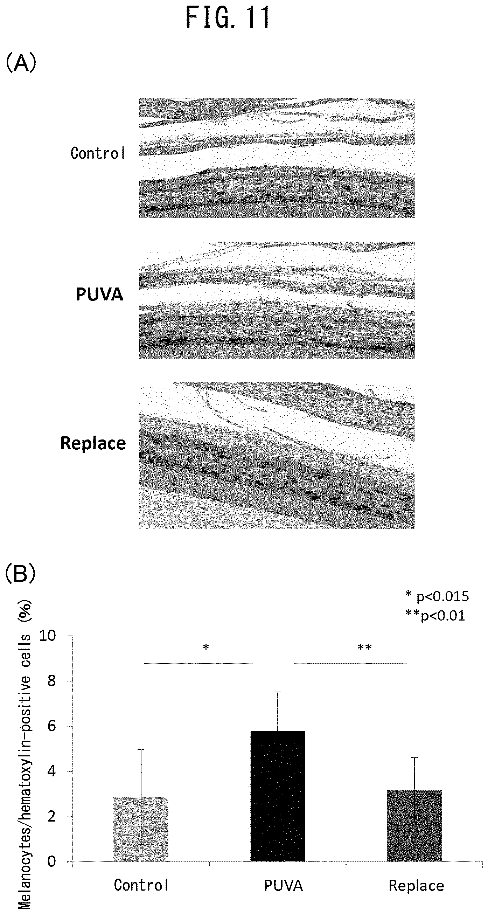

Fig. 11 shows activated melanocytes in a pigmentation skin model either treated with PUVA or not treated (control), and a PUVA-treated pigmentation epidermis model, transferred onto a PUVA-untreated dermis model and cultured (Replace). (A) Image of activated melanocytes in epidermis model slice stained using TRP2 (Tyrosine Related Protein 2) antibody, and nuclear-stained by hematoxylin staining. (B) Graph showing quantified percentage of activated melanocytes from image (A).

### DESCRIPTION OF EMBODIMENTS

Embodiments for carrying out the invention will be described in detail below with reference to the accompanying drawings, but the technical scope of the invention is not limited only to these embodiments.

The terms "first, "second" and "third" throughout the present specification are used to distinguish one element from another, and a first element may be referred to as "second element", or similarly a second element may be referred to as "first element", without deviating from the gist of the invention.

Unless otherwise defined, the terms (technical and scientific terms) used herein have the same meanings as generally understood by those skilled in the art.

### <Pigmentation skin model and method for its production>

Fig. 1 is a schematic diagram illustrating a pigmentation skin model 1 and a method for its production, according to an embodiment of the invention. For this embodiment the pigmentation skin model 1 comprises:
a first cell group 100 that includes fibroblasts that have been damaged by photoirradiation, seeded on a first cell culturing substrate 11; and
a second cell group that includes melanocytes and keratinocytes 200, applied onto the first cell group 100 (see Fig. 1(D), for example).

The pigmentation skin model 1 of this embodiment can be provided by a method that comprises the following steps:
(1) culturing the first cell group 100 that includes fibroblasts that have been damaged by photoirradiation,
(2) culturing the first cell group 100 obtained in step (1) on the first cell culturing substrate 11, and
(3) applying the second cell group 200 that includes melanocytes and keratinocytes on the first cell group 100 obtained in step (2), and culturing it.

Throughout the present specification, the structure of the pigmentation skin model 1 comprising the first cell group 100 and third cell group 100a may be referred to as "dermis model" (first dermis model or second dermis model) since it models the structure of the skin dermis. Also throughout the present specification, the structure comprising the pigmentation skin model 1 containing the second cell group 200 may be referred to as "epidermis model" since it models the structure of the skin epidermis.

The pigmentation skin model 1 may have a structure with the second cell group 200 directly seeded or layered on the first cell group 100, or it may have a structure as shown in Fig. 1, having a space formed between the second cell group 200 and first cell group 100, in which medium is present. In the latter case, the second cell group 200 is preferably seeded on a porous membrane (corresponding to the second porous membrane 210 of Fig. 1) in such a manner that liquid components secreted by the first cell group 100 and second cell group 200 can mutually pass through.

In step (3) of the method of producing the pigmentation skin model 1 according to one embodiment, the step of applying the second cell group 200 that includes melanocytes and keratinocytes onto the first cell group 100 obtained in step (2) may be a step of directly seeding or layering the second cell group 200 on the first cell group 100, or it may be a step in which the second cell group 200 that has been seeded on a second cell culturing substrate 21 having a porous membrane is applied onto the first cell group 100.

In the pigmentation skin model 1, the first cell culturing substrate 11 on which the first cell group 100 is seeded may employ a known culture substrate that allows culturing of fibroblasts, but it is preferably a cell culturing substrate having a porous membrane, and more preferably a cell culture insert. If the first cell culturing substrate 11 has a first porous membrane 110 it will be possible to supply nutrients and oxygen both above and below the cells that have been seeded on the first porous membrane 110.

In the pigmentation skin model 1 of one embodiment, the second cell culturing substrate 21 on which the second cell group 200 is seeded may employ a known culture substrate that allows culturing of melanocytes and keratinocytes, but it is preferably a cell culturing substrate having a porous membrane, and more preferably a cell culture insert. If the second cell culturing substrate 21 has a second porous membrane 210 it will be possible not only to supply nutrients and oxygen both above and below the cells that have been seeded on the second porous membrane 210, but also to allow mutual passage of liquid components secreted by the first cell group 100 and second cell group 200. If the second cell culturing substrate 21 has a second porous membrane 210 it will also be possible to separate the dermis model and epidermis model during the course of maintenance culturing of the pigmentation skin model 1, allowing culturing of each under different conditions and allowing the epidermis model to be transferred onto a different dermis model and cultured. Since formation of the epidermis layer by the epidermal cell group is affected by the quality of the dermis model, the quality of the epidermis model in the pigmentation skin model 1 can be controlled by replacement with a dermis model having the desired quality.

The mean pore size of the first porous membrane 110 and second porous membrane 210 (also applicable for the third porous membrane 110a mentioned below) may be selected as appropriate, and may be a mean pore size of about 0.01 µm to about 100 µm, for example (such as 0.01 µm to 100 µm, 0.01 µm to 50 µm, 0.01 µm to 10 µm, 0.1 µm to 50 µm or 0.1 µm to 10 µm). The density of the pores in the first porous membrane 110 and second porous membrane 210 (also applicable for the third porous membrane 110a mentioned below) may also be selected as appropriate, but it is preferably a pore density of 1 × 10⁴/cm² or greater, 1 × 10⁵/cm² or greater, 5 × 10⁵/cm² or greater, 10 × 10⁵/cm² or greater, 50 × 10⁵/cm² or greater or 100 × 10⁵/cm² or greater, and the range may be 1 × 10⁴/cm² to 100 × 10⁸/cm² or 1 × 10⁵/cm² to 100 × 10⁸/cm².

According to one embodiment, if the second cell culturing substrate 21 is a cell culture insert, the second cell culturing substrate 21 (cell culture insert) used has a smaller inner diameter than the first cell culturing substrate 11. This will allow the second cell culturing substrate 21 to be used by insertion into the culturing portion of the first cell culturing substrate 11.

The cells used for the invention may be derived from any animal, but they are preferably derived from a vertebrate, more preferably from a mammal, and most preferably from a human.

Fibroblasts are one of the types of cells constituting connective tissue, and they are present in a large number of organs and tissues. Fibroblasts are included in skin, and primarily the dermal tissue. The fibroblasts to be used for the pigmentation skin model 1 of the invention are preferably fibroblasts derived from the dermis.

Keratinocytes are a type of cells composing the epidermis, and in biological epidermal tissue they divide at the deepest part (basal lamina) while migrating to the surface and differentiating toward the upper layer into the stratum spinosum, granular layer and stratum corneum, eventually being shed as old skin.

Melanocytes are a type of cells composing epidermal tissue, which are present in the epidermal basal lamina of biological tissue and produce melanin.

The fibroblasts, keratinocytes and melanocytes used for the invention may each be primary cultured cells harvested from biological tissue, or cells marketed or distributed after first having been isolated and/or grown, or cells of an established cell line, or cells differentiated from pluripotent stem cells such as ES cells, iPS cells or Muse cells.

The first cell group 100 may also contain cells other than fibroblasts, such as mast cells, histiocytes, plasmocytes or dermal dendritic cells that are present within dermal tissue. The number of fibroblasts in the first cell group 100 may be 1 × 10⁴ to 10⁸/cm², and preferably 0.1 to 10 × 10⁵/cm².

The second cell group 200 may also include cells other than the keratinocytes and melanocytes, such as Langerhans cells in the epidermal tissue, or Merkel cells. The second cell group 200 may include the cells in a proportion of keratinocytes:melanocytes of 1:1 to 1000:1 and preferably 3:1 to 30:1. The second cell group 200 includes the keratinocytes at 1 × 10² to 10⁶/cm², preferably 1.0 to 10 × 10⁴/cm² and more preferably about 4 to 8 × 10⁴/cm², and the melanocytes at 1 to 10 × 10³/cm² and preferably 4 to 8 × 10³/cm².

The fibroblasts used in the first cell group 100 are fibroblasts that have been damaged by photoirradiation. The fibroblasts damaged by photoirradiation are preferably ones that have been damaged by photoirradiation in the presence of a photosensitizer. Examples of photosensitizers to be used include psoralen, NAD, riboflavin, tryptophan, folic acid, porphyrin, methylene blue, and thiol group-protected gold nanoclusters (AUxSRy). By using a photosensitizer it is possible to cause sensitization to the irradiated light and more efficiently induce damage in the fibroblasts.

The light used to induce damage in the fibroblasts may have a wavelength which causes damage in intracellular nucleic acid such as DNA or RNA but does not result in death of all of the cells, and it is preferably ultraviolet light (about 200 nm to about 400 nm), and more preferably UVA (about 320 nm to about 400 nm). The intensity of the irradiated light may be such as to produce damage in the intracellular nucleic acid such as DNA or RNA while not inducing apoptosis and death in all of the cells, and it can be appropriately adjusted by the wavelength or irradiation time, and the cell density. Irradiation of UVA, for example, may be at 0.01 J/cm² to 100 J/cm², preferably 0.1 J/cm² to 20 J/cm² and more preferably 0.5 J/cm² to 10 J/cm².

If the photoirradiated fibroblasts are cultured for a fixed time period it is possible to cause proliferation of only the surviving fibroblasts that have not undergone apoptosis (Fig. 1(B)). Fibroblasts that have suffered damage by photoirradiation have elongated cellular forms and reduced proliferation potency, as well as increased production of melanin-producing factors (such as stem cell growth factor (SCF), and thus an increased level of cell senescence (see Fig. 3). The level of senescence of cells can be examined by measuring expression levels of commonly known cell senescence markers such as senescent acidic β-galactosidase (SA-βgal), constitutive activation of cell cycle check mechanisms such as the p21/p53 pathway or p16 pathway, or expression levels of cell senescence-associated secretory phenotype (SASP) factors such as IL-6. Adjustment of the photoirradiation dose allows fibroblasts with the desired level of senescence to be obtained.

The fibroblasts in the first cell group 100 of the pigmentation skin model 1 are fibroblasts that have been damaged by photoirradiation but have survived without dying. The pigmentation skin model 1 of the invention therefore contains largely homogeneous fibroblasts, and can thus provide a stable system with low variability of activity.

According to one embodiment, the epidermis model 20 used to form the pigmentation skin model 1 may be TESTSKIN^{R} LSE-melano (TOYOBO) or MelanoDerm^{R} (MatTek), for example, which are commercially available.

The pigmentation skin model 1 may be cultured using a culture solution commonly used as a culture solution for culturing of keratinocytes, such as KG medium, EpilifeKG2 (Kurabo Industries, Ltd.), Humedia-KG2 (Kurabo Industries, Ltd.) or assay medium (TOYOBO), at about 37°C for a period of 0 to 14 days. The culture medium used may alternatively be DMEM culture medium (Gibco), or a culture medium comprising a 1:1 mixture of ascorbic acid-containing KGM and DMEM.

The first cell group 100 preferably is seeded together with a hydrogelling agent. As used herein, "hydrogelling agent" is a substance added in order to form a hydrogel. Hydrogelling agents to be used for the invention may be selected from the group consisting of include collagen, gelatin, hyaluronate, hyaluronan, fibrin, alginate, agarose, chitosan, chitin, cellulose, pectin, starch, laminin, fibrinogen/thrombin, fibrillin, elastin, gum, cellulose, agar, gluten, casein, albumin, vitronectin, tenascin, entactin/nidogen, glycoproteins, glycosaminoglycan, poly(acrylic acid) and its derivatives, poly(ethylene oxide) and its copolymers, poly(vinyl alcohol), polyphosphazene, Matrigel, and combinations of the foregoing.

According to one embodiment, step (2) may be carried out in the presence of ascorbic acid, an ascorbic acid derivative, or salt thereof. Ascorbic acid, an ascorbic acid derivative or salt thereof is preferably present in order to promote multilayer formation similar to the structure of the dermis, by accelerated proliferation of the fibroblasts and collagen production. Throughout the present specification, "ascorbic acid derivative" refers to ascorbic acid diphosphate, ascorbic acid monophosphate, sodium L-ascorbate or L-ascorbic acid 2-glucoside, or a salt thereof (such as a sodium salt or magnesium salt).

The pigmentation skin model 1 provided for the invention has fibroblasts damaged by photoirradiation that has acted directly or indirectly on melanocytes, accelerating melanin production. The extent of pigment production and/or pigmentation in the pigmentation skin model 1 can then be measured to evaluate factors that affect pigmentation.

### <Method for evaluating factors for treatment or prevention of skin pigmentation (first mode)>

The invention can provide a method for evaluating factors for treatment or prevention of skin pigmentation using the pigmentation skin model. According to one embodiment, the method of the invention comprises:
(1) applying a candidate factor to a pigmentation skin model 1 and culturing it, and
(2) evaluating the treatment or prevention effect of the candidate factor, using the extent of pigment production and/or pigmentation in the pigmentation skin model 1 as an indicator.

According to another embodiment, candidate factors include low molecular compounds, peptides, nucleic acids, proteins, mammalian animal (such as mouse, rat, porcine, bovine, sheep, monkey or human) cells, tissue extracts or cell culture supernatants, plant-derived compounds or extracts (such as galenical extracts or galenical-derived compounds), and microbial compounds or extracts, or culture products.

A candidate substance may be added to the pigmentation skin model 1 and culturing carried out for a predetermined time period, and then the extent of pigment production and/or pigmentation in the pigmentation skin model 1 may be measured to evaluate the treatment or prevention effect that the candidate substance has on pigmentation. For example, it is possible to compare the extent of pigment production and/or pigmentation in a pigmentation skin model 1 without addition of a candidate substance, and with addition of a substance that does not have a treatment or prevention effect on pigmentation, and when the extent of pigment production and/or pigmentation in the pigmentation skin model 1 with the added candidate substance is higher, the candidate substance may be evaluated as having a treatment or prevention effect on pigmentation.

As used herein, "pigment production" refers to production of a pigment such as melanin pigment, which is produced by the pigmentation skin model. Melanin pigment is produced primarily by melanocytes. The amount of pigment production can be determined as the amount of melanin, by extracting the melanin pigment from the pigmentation skin model, and especially the second cell group (keratinocytes and melanocytes), and measuring the absorbance at 405 nm. The amount of pigment production can be measured as the level of melanin or nucleic acid (such as mRNA) coding therefor, present in the pigmentation skin model and particularly the second cell group (keratinocytes and melanocytes), using a method such as ELISA, flow cytometry, Western blotting, an immunohistochemical method or qPCR, with no limitation to these.

As used herein, "extent of pigmentation" means the color brightness of the pigmentation skin model, and particularly the second cell group (keratinocytes and melanocytes), under visible light. The pigmentation skin model of the invention has varying brightness depending on the amount of melanin produced by the melanocytes. A greater amount of melanin results in reduced brightness, so that the pigmentation skin model exhibits a darker color. A lower amount of melanin, conversely, increases the brightness and causes the pigmentation skin model to exhibit a lighter color. That is, the treatment or prevention effect on pigmentation by an added candidate substance can be evaluated by comparing the color brightness of the pigmentation skin model. The brightness can be quantified by recording the pigmentation skin model as an image and using publicly known image measuring means.

### <Method for evaluating factors for treatment or prevention of skin pigmentation (second mode)>

According to another embodiment, the method of the invention comprises:
(1) culturing a first cell group 100 that includes fibroblasts that have been damaged by photoirradiation,
(2) culturing the first cell group 100 obtained in step (1) on a first cell culturing substrate 11,
(3) applying a second cell group 200 that includes melanocytes and keratinocytes onto the first cell group 100 obtained in step (2) and culturing it,
(4) applying the second cell group obtained in step (3) onto a substrate containing a candidate factor, and culturing the resulting pigmentation skin model 1a, and
(5) evaluating the treatment or prevention effect of the candidate factor, using the extent of pigment production and/or pigmentation in the pigmentation skin model 1a as an indicator.

This other embodiment will now be explained with reference to Fig. 2. Steps (1) to (3) are the same as the steps described for the method for producing a pigmentation skin model and will not be explained again here (Fig. 2(A) to (D)). Likewise, step (5) is the same as step (2) described above for "Method for evaluating factors for treatment or prevention of skin pigmentation" (first mode) and will not be explained again here.

According to one embodiment, the invention comprises applying the second cell group 200 obtained in step (3) onto a substrate containing a candidate factor, and culturing the obtained pigmentation skin model 1a (step (4), Fig. 2(F)). The candidate factor on the substrate used in step (3) (such as the third cell culturing substrate 11a in Fig. 2(E) and (F)) may be, for example, a low molecular compound, peptide, nucleic acid or protein, a mammalian animal (such as mouse, rat, porcine, bovine, sheep, monkey or human) cells, a tissue extract or cell culture supernatant, plant-derived compound or extract (such as a galenical extract or galenical-derived compound), a microbial compound or extract, or a culture product. According to one embodiment, as shown in Fig. 2(E) and (F), the candidate factor may be any type of cells, such as fibroblast progenitor cells including mesenchymal stem cells, or fibroblasts (such as fibroblasts that have not been damaged by photoirradiation, or fibroblasts with low damage by photoirradiation). Throughout the present specification, "fibroblasts that have not been damaged by photoirradiation" or "fibroblasts with low damage by photoirradiation" are fibroblasts that have not been subjected to a photoirradiation step, and that have a lower level of senescence than the "fibroblasts that have been damaged by photoirradiation" mentioned above. According to one embodiment, the third cell group 100a used as the candidate factor may be one that has been seeded on the third cell culturing substrate 11a having a porous membrane.

According to another embodiment, step (4) may be directly seeding or layering the second cell group 200 obtained in step (3) onto the third cell group 100a used as the candidate factor, or it may be applying the second cell group 200 that has been seeded on the second cell culturing substrate 21 having a porous membrane, onto the third cell group 100a used as the candidate factor. In the latter case it is possible to easily transfer the second cell culturing substrate 21 onto another dermis model.

### EXAMPLES

The present invention will now be explained in greater detail by examples, with the understanding that the invention is not limited in any way by the examples.

### 1. Materials and methods used

### 1-1. Fibroblast culturing and PUVA treatment

Normal human fibroblasts (1 × 10⁵ cells) were cultured with a growth medium (DMEM + 10% fetal calf serum). Before reaching 100% confluence, the medium was exchanged with growth medium containing psoralen (final concentration: 25 ng/mL) (Sigma-Aldrich), and culturing was continued. After 24 hours, the cells were imbibed with 1 mL phosphate-buffered saline (PBS), the PBS was removed, the medium was exchanged with 1 mL PBS containing psoralen (final concentration: 25 ng/mL), and UVA irradiation (SAN-EI UVE-502S) was carried out at 6 J/cm² (hereunder this will be referred to as "PUVA treatment"). This was followed by imbibing with 1 mL PBS and then removal of the PBS, exchange with growth medium, and continued culturing for about 2 to 7 days. Some of the cells died by the effects of PUVA treatment during the procedure, with the surviving fibroblasts continuing to proliferate. Fibroblasts from the same donor without PUVA treatment were used as a control in comparison with the PUVA-treated fibroblasts (Figs. 3 to 6).

### 1-2. Preparation of dermis model

A dermis model was prepared using fibroblasts subjected to the PUVA treatment of 1-1. above, or without PUVA treatment (control). For cursory examination, the procedure of 1. above was followed by removal of the growth medium and imbibing with 1 mL PBS. The PBS was then removed, a cell release agent (TrypLE SELECT, Thermo Fisher Scientific) was added at 300 µL per well of a 6-well plate, and the mixture was allowed to stand for 5 minutes in a 5% CO₂ incubator at 37°C as cell releasing treatment. Growth medium was added to suspend the reaction, and the cell suspension was recovered in a 15 ml centrifuge tube. After centrifugation at 1000 rpm for 5 minutes, the supernatant was removed and the cell pellet was resuspended in growth medium and adjusted to 5 × 10⁵ cells/mL. A gel suspension comprising the composition listed in Table 1 was prepared on ice to prepare a dermis model for application to each well of a 6-well plate.

### [Table 1]

**Table 1: Composition of gel suspension for dermis model containing fibroblasts**

| Reagent | Amount |
|---|---|
| Pig skin collagen (final concentration: 0.5% (w/v)) (NH Foods Ltd., 309-31595) | 1.5 mL |
| 5 × DMEM | 0.6 mL |
| Fetal bovine serum (final concentration: 10% (v/v)) | 0.3 mL |
| L-ascorbic acid 2-glucoside(AA2G) (final concentration: 250 µM) (Sigma-Aldrich, SMB00390) | 3 µL |
| Fibroblast suspension | 0.6 mL |
| Total volume | 3 mL |

A cell culture insert for dermis model preparation was set in the 6-well plate and 3 mL of gel suspension was added into the cell culture insert. After solidifying the gel suspension with a 5% CO₂ incubator at 37°C, 2 mL of growth medium containing ascorbic acid (AA2G) was added to the 6-well plate and culturing was carried out for 24 hours in the 5% CO₂ incubator at 37°C.

### 1-3. Preparation of pigmentation skin model combined with epidermis model

Separately from the dermis model, a commercially available epidermis model (MatTek Co.) comprising melanocytes and keratinocytes was also used (see Fig. 1(C)).

The prepared epidermis model was laid over and combined with the dermis model of 1-2. above (see Fig. 1(D)) and set in a specialized vessel (Corning BioCoat deep well plate, 6 wells) (Corning #355467), and then 9.5 mL of three-dimensional skin model medium (a 1:1 mixture of epidermis model special medium (MatTek #EPI-100-NMM-113) and DMEM) was added and medium exchange was carried out with the same medium at a frequency of once every 3 to 4 days.

### 1-4. Pigmentation skin model with replaced dermis model

An epidermis model cultured for 10 days in combination with a dermis model prepared using PUVA-treated fibroblasts was replaced with a dermis model prepared using PUVA-untreated normal fibroblasts, and culturing was continued for 10 days (see Fig. 2(E) and (F)). The replacement dermis model (dermis model prepared using PUVA-untreated fibroblasts) was prepared on the day prior to replacement.

### 2. Results

### 2-1. PUVA-treated fibroblasts

Fig. 3 shows PUVA-treated and untreated fibroblasts. The PUVA-treated fibroblasts had become elongated (Fig. 3(A)). Upon examining variation in the number of proliferated PUVA-treated and untreated fibroblasts, it was found that the PUVA-treated fibroblasts had a notably lower growth rate (Fig. 3(B)).

### 2-2. Senescence level of PUVA-treated fibroblasts

Upon examining the enzyme activity values of senescence-associated factor SA-β-gal in cell lysates of the PUVA-treated and untreated fibroblasts, it was found that the PUVA-treated fibroblasts had notably increased SA-β-gal enzyme activity (Fig. 4(A)). With SA-β-gal staining, the PUVA-treated fibroblasts were observed to have a very large number of SA-β-gal positive cells (blue) (Fig. 4(B)).

### 2-3. Production level of melanin-producing factor SCF in PUVA-treated fibroblasts

When PUVA-treated and untreated fibroblasts were each cultured by monolayer culture, with measurement of the SCF amounts in the culture supernatants 1 day before PUVA treatment and 0, 1, 3, 7, 14 and 21 days after PUVA treatment by ELISA, and calculation of the amount of secretion per cell, the SCF level of the PUVA-treated fibroblasts was found to be notably higher (Fig. 5(A)). When SCF staining with SCF antibody and nuclear staining with DAPI were carried out and a fused (merged) image for the SCF staining and nuclear stain was created, a large number of SCF-positive cells were observed among the PUVA-treated fibroblasts (Fig. 5(B)).

### 2-4. Production level of melanin-producing factor HGF in PUVA-treated fibroblasts

When PUVA-treated and untreated fibroblasts were each cultured by monolayer culture, and then HGF staining with HGF antibody and nuclear staining with DAPI were carried out and a fused (merged) image for the HGF stain and nuclear stain was created, a large number of HGF-positive cells were observed among the PUVA-treated fibroblasts (Fig. 6(A)).

### 2-5. Production level of melanin-producing factor HGF in PUVA-treated fibroblasts

Fig. 7 is a diagram showing pigmentation in an epidermis model, for a pigmentation skin model either with PUVA treatment (A) or without treatment (B). The epidermis model for the PUVA-treated pigmentation skin model showed accelerated pigmentation.

### 2-6. Pigmentation skin model with replaced dermis model

An epidermis model cultured for 10 days in combination with a dermis model prepared using PUVA-treated fibroblasts was replaced with a dermis model prepared using PUVA-untreated normal fibroblasts, and after culturing for another 10 days, pigmentation was observed to be inhibited (Fig. 8) compared to pigmentation in the epidermis model for the PUVA-treated pigmentation skin model (Fig. 7(A)).

### 2-7. Pigmentation in pigmentation skin models with PUVA treatment, without treatment, and with dermis model replacement

A PUVA-treated or untreated (control) pigmentation skin model, and an epidermis model which was an epidermis model of a PUVA-treated pigmentation skin model transferred onto a PUVA-untreated dermis model and cultured (Replace), were outwardly observed, and as a result the pigmentation in the epidermis model of the PUVA-treated pigmentation skin model was found to be accelerated while pigmentation in the epidermis model under the Replace conditions was alleviated (Fig. 9(A)). Upon quantification based on the image of Fig. 9(A), the ratio of the thickest pigmented region in the PUVA-treated pigmentation skin model was high, while the region of thick pigmentation in the pigmentation skin model under the Replace conditions was reduced (Fig. 9(B)).

### 2-8. Melanin formation in pigmentation skin model with PUVA treatment, without treatment, and with dermis model replacement

After preparing slices of a PUVA-treated or untreated (control) pigmentation skin model, and an epidermis model which was a PUVA-treated pigmentation skin model replaced on a PUVA-untreated dermis model and cultured (Replace), the melanin granules in the epidermis model slices were stained by Fontana-Masson staining (Fig. 10(A)). When the stained image of Fig. 10(A) was binarized and the ratio of melanin granule regions was quantified, it was found that the ratio of melanin regions was highest in the PUVA-treated pigmentation skin model, while the melanin regions in the pigmentation skin model under the Replace conditions were reduced (Fig. 10(B)).

### 2-9. Number of melanocytes in pigmentation skin model with PUVA treatment, without treatment, and with dermis model replacement

After preparing slices of a PUVA-treated or untreated (control) pigmentation skin model, and an epidermis model which was a PUVA-treated pigmentation skin model replaced on a PUVA-untreated dermis model and cultured (Replace), TRP2 antibody was used for staining of the activated melanocytes in the epidermis model slices, and the nuclei were stained by hematoxylin staining (Fig. 11(A)). When the ratio of activated melanocytes was quantified from the image of Fig. 11(A), it was found that the ratio of activated melanocytes was highest in the PUVA-treated pigmentation skin model, while the number of activated melanocytes in the pigmentation skin model under the Replace conditions was reduced (Fig. 11(B)).

### REFERENCE SIGNS LIST

1, 1a Pigmentation skin model
10 First dermis model
100 First cell group
11 First cell culturing substrate
110 First porous membrane
12 First cell culturing vessel
13 First medium
14 Second medium
10a Second dermis model
100a Third cell group
11a Third cell culturing substrate
110a Third porous membrane
20 Epidermis model
200 Second cell group
21 Second cell culturing substrate
210 Second porous membrane
22 Second cell culturing vessel
23 Third medium
24 Fourth medium
RD Photoirradiation
FB Fibroblasts
bFB Fibroblasts damaged by photoirradiation
KC Keratinocytes
MC Melanocytes

## Claims

1. A pigmentation skin model comprising:
a first cell group that includes fibroblasts that have been damaged by photoirradiation, seeded on a first cell culturing substrate; and
a second cell group that includes melanocytes and keratinocytes, applied onto the first cell group.

2. The pigmentation skin model according to claim 1, wherein the fibroblasts that have been damaged by photoirradiation are fibroblasts that have been damaged by irradiation of ultraviolet light in the presence of a photosensitizer.

3. The pigmentation skin model according to claim 2, wherein the photosensitizer is selected from the group consisting of psoralen, NAD, riboflavin, tryptophan, folic acid, porphyrin, methylene blue and thiol group-protected gold nanoclusters (AUxSRy).

4. The pigmentation skin model according to any one of claims 1 to 3, wherein the photoirradiation is photoirradiation with UVA.

5. The pigmentation skin model according to any one of claims 1 to 4, wherein the second cell group is a second cell group seeded onto a second cell culturing substrate having a porous membrane.

6. The pigmentation skin model according to any one of claims 1 to 5, wherein the first cell culturing substrate is a cell culturing substrate having a porous membrane.

7. The pigmentation skin model according to any one of claims 1 to 6, wherein the first cell group is a first cell group seeded together with a hydrogelling agent.

8. The pigmentation skin model according to claim 7, wherein the hydrogelling agent is selected from the group consisting of collagen, gelatin, hyaluronate, hyaluronan, fibrin, alginate, agarose, chitosan, chitin, cellulose, pectin, starch, laminin, fibrinogen/thrombin, fibrillin, elastin, gum, cellulose, agar, gluten, casein, albumin, vitronectin, tenascin, entactin/nidogen, glycoproteins, glycosaminoglycan, poly(acrylic acid) and its derivatives, poly(ethylene oxide) and its copolymers, poly(vinyl alcohol), polyphosphazene, Matrigel, and combinations of the foregoing.

9. A method for producing a pigmentation skin model, wherein the method comprises:
(1) culturing a first cell group that includes fibroblasts that have been damaged by photoirradiation,
(2) culturing the first cell group obtained in step (1) on a first cell culturing substrate, and
(3) applying a second cell group that includes melanocytes and keratinocytes on the first cell group obtained in step (2), and culturing it.

10. The method according to claim 9, wherein the fibroblasts that have been damaged by photoirradiation are fibroblasts that have been damaged by irradiation of ultraviolet light in the presence of a photosensitizer.

11. The method according to claim 10, wherein the photosensitizer is selected from the group consisting of psoralen, NAD, riboflavin, tryptophan, folic acid, porphyrin, methylene blue and thiol group-protected gold nanoclusters (AUxSRy).

12. The method according to any one of claims 9 to 11, wherein the photoirradiation is photoirradiation with UVA.

13. The method according to any one of claims 9 to 12, wherein the second cell group is a second cell group seeded onto a second cell culturing substrate having a porous membrane.

14. The method according to any one of claims 9 to 13, wherein the first cell culturing substrate is a cell culturing substrate having a porous membrane.

15. The method according to any one of claims 9 to 14, wherein the first cell group is a first cell group seeded together with a hydrogelling agent.

16. The method according to claim 15, wherein the hydrogelling agent is selected from the group consisting of collagen, gelatin, hyaluronate, hyaluronan, fibrin, alginate, agarose, chitosan, chitin, cellulose, pectin, starch, laminin, fibrinogen/thrombin, fibrillin, elastin, gum, cellulose, agar, gluten, casein, albumin, vitronectin, tenascin, entactin/nidogen, glycoproteins, glycosaminoglycan, poly(acrylic acid) and its derivatives, poly(ethylene oxide) and its copolymers, poly(vinyl alcohol), polyphosphazene, Matrigel, and combinations of the foregoing.

17. The method according to any one of claims 9 to 16, wherein the step (2) is carried out in the presence of ascorbic acid, an ascorbic acid derivative or salt thereof.

18. A pigmentation skin model obtained by the method according to any one of claims 9 to 17.

19. A method for evaluating factors for treatment or prevention of skin pigmentation, wherein the method comprises:
(1) applying a candidate factor to a pigmentation skin model according to any one of claims 1 to 8 or 18, and culturing it, and
(2) evaluating the treatment or prevention effect of the candidate factor, using the extent of pigment production and/or pigmentation in the pigmentation skin model as an indicator.

20. A method for evaluating factors for treatment or prevention of skin pigmentation, wherein the method comprises:
(1) culturing a first cell group that includes fibroblasts that have been damaged by photoirradiation,
(2) culturing the first cell group obtained in step (1) on the first cell culturing substrate,
(3) applying a second cell group that includes melanocytes and keratinocytes onto the first cell group obtained in step (2) and culturing it,
(4) transferring the second cell group obtained in step (3) onto a substrate containing a candidate factor, and culturing the pigmentation skin model, and
(5) evaluating the treatment or prevention effect of the candidate factor, using the extent of pigment production and/or pigmentation in the pigmentation skin model as an indicator .

21. The method according to claim 20, wherein the candidate factor is fibroblasts.
